# EUROPEAN PATENT SPECIFICATION

(11) **EP 1 844 718 B1**
(45) Date of publication and mention of the grant of the patent: **24.12.2014**
(21) Application number: 06712711.8
(22) Date of filing: 31.01.2006
(51) Int. Cl.: A61B 17/12, A61B 17/04

(54) **MEDICAL SUTURE AND LIGATURE INSTRUMENT AND MEDICAL SUTURE AND LIGATURE TOOL**
MEDIZINISCHES NAHT- UND LIGATIONSINSTRUMENT UND MEDIZINISCHES NAHT- UND LIGATIONSWERKZEUG
DISPOSITIF MEDICAL DE SUTURE ET LIGATURE ET OUTIL MEDICAL DE LIGATURE ET SUTURE

(30) Priority: 04.02.2005 JP 2005028676
(43) Date of publication of application: 17.10.2007
(73) Proprietor: Olympus Corporation, Tokyo 151-0072 (JP)
(72) Inventor: MIYAMOTO, Satoshi c/o OLYMPUS CORPORATION, Shibuya-ku, Tokyo (JP); ONISHI, Norio, Tokyo, 1970012 (JP)
(74) Representative: von Hellfeld, Axel
(86) International application number: PCT/JP2006/301568
(87) International publication number: WO 2006/082810

(56) References cited:
- WO-A1-01/89393
- JP-A- 2003 204 966
- JP-A- 2004 000 601
- US-A1- 2003 144 673
- US-A1- 2003 236 535

## Description

### TECHNICAL FIELD

The present invention relates to a medical suture and ligature instrument and a medical suture and ligature tool which are used along with an endoscope and used for an endoscopic treatment such as suturing and ligaturing a biological tissue in a body

This application claims the priority of Japanese Patent Application JP 2006 212 240 A filed on February 4, 2005.

### BACKGROUND ART

A medical suture and ligature instrument for suturing or ligaturing a biological tissue in a body while observing the biological tissue by the use of an endoscope is known, which includes a sheath to be inserted through a channel of the endoscope, an operating wire inserted into the sheath to extend and retract, and a medical suture and ligature tool to be locked to a hook at a distal end of the operating wire. The medical ligature tool has a ligature wire of a loop shape and the size of the loop can be adjusted by allowing a stopper to extend and retract along the ligature wire. A folding-back portion to be locked to the hook is formed in the proximal end portion of the ligature wire. At the time of ligaturing a pathological lesion portion, the loop is hooked to the pathological lesion portion, the hook is made to retract, and the diameter of the loop is reduced, thereby binding the pathological lesion portion tight. Thereafter, the ligature wire is cut between the portion binding the pathological lesion portion tight and the folding-back portion and the ligature wire is detained in the body in a state where the biological tissue is bound tight.

Here, a medical ligature tool is also known in which a cutting member is disposed outside of a sheath into which an operating wire is inserted so as to extend and retract and a holding member for receiving the cutting member while holding the ligature wire between a folding-back portion and a stopper is disposed in the medical ligature tool, so as to perform as a series of operations an operation of tightly binding and ligaturing a pathological lesion portion with the medical ligature tool and an operation of separating the medical ligature tool from the operating wire (for example, see Patent Document 1). The holding member has a cylindrical shape through which the ligature wire is inserted and an annular protrusion is incorporated in a substantially central portion in an axial direction thereof. The portions closer to the distal and the proximal ends with respect to the protrusion each have a hole through which the ligature wire can be inserted. The ligature wire is inserted inside via a distal opening of the passage penetrating the holding member, passes through the distal hole, is drawn along the outer periphery of the protrusion in the longitudinal direction, extends into the holding member through the proximal hole, and then is drawn out of the proximal opening. In such a medical ligature tool, a pathological lesion portion is tightly bound, the cutting member is made to advance, and the ligature wire is cut off by interposing the ligature wire between the protrusion and the cutting member. The holding member spontaneously falls off and is discharged after the ligature wire is cut.

[Patent Document 1] Japanese Unexamined Patent Publication No. 2003-204966.

US 2003/236535 A1 discloses a ligating unit comprising a thread, a holding member composed of two members, and a cutter.

### DISCLOSURE OF THE INVENTION

### Problems that the Invention is to Solve

However, in the conventional medical ligature tool, when the friction between the holding member and the ligature wire is large, the holding member might not spontaneously fall off. In this case, it is necessary to grasp the holding member with an endoscopic treatment tool such as grasping forceps and to separate the holding member from the ligature wire, so as not to allow the holding member to interfere with other treatments. In this case, since it is necessary to pull out the medical suture and ligature instrument via a channel of an endoscope and then to insert the endoscopic treatment tool such as the grasping forceps instead, there is a problem in that surgical procedure becomes complicated and operation time is prolonged.

The invention is contrived to solve the above-mentioned problem. An object of the invention is to reliably separate the holding member after suturing or ligaturing a biological tissue and cutting a suture and ligature member such as a ligature wire.

### Means for Solving the Problems

A medical suture and ligation tool according to claim 1 is provided.

### Advantages of the Invention

According to the present invention, since the holding member for holding the suture and ligature member includes a plurality of members, a target part is sutured or ligatured under the endoscopic observation, an extra suture and ligature member is cut, and then a plurality of members constituting the holding member is separated or a part thereof is moved, whereby the suture and ligature member can be easily pulled out. Accordingly, it is possible to reliably and rapidly separate the holding member from the suture and ligature member. As a result, it is not necessary to pull out the holding member from the suture and ligature member by the use of another treatment tool, thereby a surgical procedure can be performed easily with a short time.

### BRIEF DESCRIPTION OF THE DRAWINGS

FIG. 1 is a diagram illustrating a configuration of a medical ligature instrument according to an example.
FIG. 2 is an exploded sectional view of a holding member.
FIG. 3 is a diagram illustrating a state where a medical ligature tool is fitted to an operation unit and is received in an outer sheath.
FIG. 4 is a diagram illustrating a procedure of ligaturing a pathological lesion portion, which shows a state where a ligature wire is hooked to the pathological lesion portion.
FIG. 5 is a cross-sectional view illustrating a state where a diameter of a loop portion of the ligature wire is reduced by drawing back an operating wire.
FIG. 6 is an enlarged sectional view illustrating a state where the ligature wire is cut.
FIG. 7 is a diagram illustrating a procedure of ligaturing a pathological lesion portion, which shows a state where the ligature wire is cut.
FIG. 8 is an enlarged sectional view illustrating a state where a first cylindrical member is separated.
FIG. 9 is a diagram illustrating a procedure of ligaturing a pathological lesion portion, which shows a state where the first cylindrical member is separated.
FIG. 10 is a diagram illustrating a state where the first and second cylindrical members are both separated.
FIG. 11 is a cross-sectional view illustrating a distal end portion of a medical suture instrument according to an example.
FIG. 12 is a diagram illustrating a state where a biological tissue is punctured with a needle portion of a treatment instrument.
FIG. 13 is a diagram illustrating where a separation-preventing tip of the medical suture tool is pushed out of the needle portion.
FIG. 14 is a diagram illustrating a state where the needle portion is pulled out of the biological tissue and a suture thread is detained therein.
FIG. 15 is a diagram illustrating a procedure of suturing the biological tissue by drawing the suture thread.
FIG. 16 is a diagram illustrating a state where the biological tissue is sutured with the suture thread.
FIG. 17 is a diagram illustrating an example where a biological tissue is sutured by the use of a plurality of medical suture tool.
FIG. 18 is a perspective view illustrating a configuration of a holding unit.
FIG. 19 is a perspective view illustrating a first member constituting the holding unit.
FIG. 20 is a perspective view illustrating a configuration of a holding unit.
FIG. 21 is an enlarged sectional view illustrating a state where the ligature wire is cut.
FIG. 22 is a diagram illustrating a state where a second cylindrical member slides to alter the length of a distal lateral hole.

### DESCRIPTION OF REFERENCE NUMERALS AND SIGNS

1: MEDICAL SUTURE AND LIGATURE INSTRUMENT
2: MEDICAL LIGATURE TOOL (MEDICAL SUTURE AND LIGATURE TOOL)
3,51: OPERATION UNIT
4: INSERTION SECTION
10: CUTTING MEMBER
12: HANDLE OPERATING SECTION
20: LIGATURE WIRE (SUTURE AND LIGATURE MEMBER)
22: STOPPER (FIXING MEMBER)
23, 63, 70, 90, 100: HOLDING MEMBER
30, 64: FIRST CYLINDRICAL MEMBER (MEMBER)
31, 65: SECOND CYLINDRICAL MEMBER (MEMBER)
37a, 37b, 103a, 103b: LATERAL HOLE FORMING PORTION
42a, 42b, 79a, 79b, 106a, 106b: DISTAL LATERAL HOLE (LATERAL HOLE)
50: MEDICAL SUTURE INSTRUMENT (MEDICAL SUTURE AND LIGATURE INTRUMENT)
52: MEDICAL SUTURE TOOL (MEDICAL SUTURE AND LIGATURE TOOL)
53: MEDICAL PUNCTURE INSTRUMENT
56: NEEDLE PORTION (TISSUE PUNCTURE MEMBER)
59: SEPARATION-PREVENTING TIP (LOCKING MEMBER)
61: SUTURE THREAD (SUTURE AND LIGATURE MEMBER)
74a, 74b, 75a, 75b, 77a, 77b, 78a, 78b: NOTCH PORTION (LATERAL HOLE FORMING PORTION)
80a, 80b: PROXIMAL LATERAL HOLE (LATERAL HOLE)
101: FIRST MEMBER
102: SECOND MEMBER
W1: PATHOLOGICAL LESION PORTION (BIOLOGICAL TISSUE)
W2, W3: BIOLOGICAL TISSUE

### BEST MODE FOR CARRYING OUT THE INVENTION

Examples and a preferred embodiment of the invention will be described in detail with reference to the drawings. However, the invention is not limited to the following embodiment, but, for example, elements of the examples and the embodiment may be properly combined.

### First example

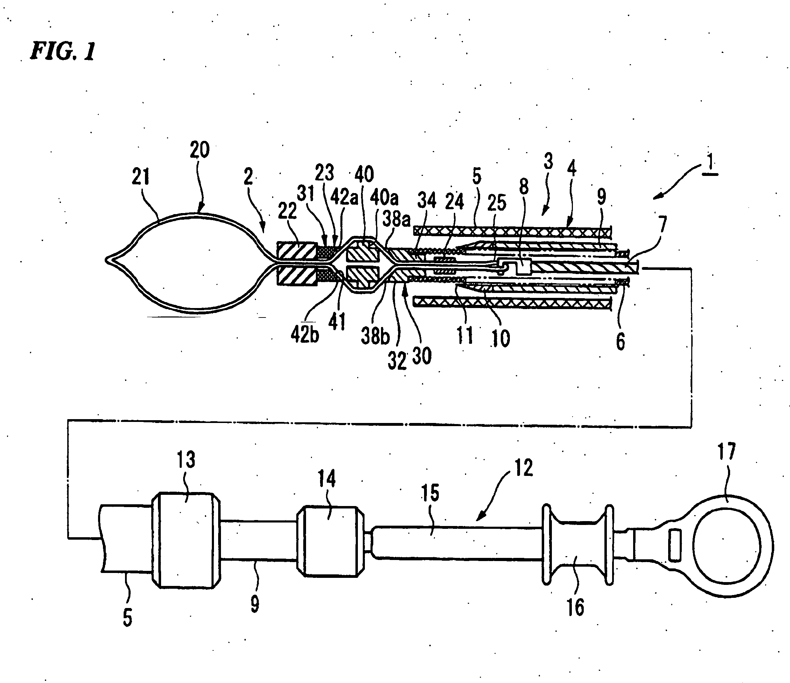

Hereinafter, a first example will be described with reference to FIGS. 1 to 8. As shown in FIG. 1, a medical suture and ligature instrument 1 includes a medical ligature tool (medical suture and ligature tool) 2 which is detained in a body and an operation unit 3 which allows the medical ligature tool 2 to be detachably attached to the distal end thereof. The operation unit 3 has an elongated insertion portion 4 of which the distal end is inserted into a channel of an endoscope (not illustrated) and of which the proximal end is drawn out via the endoscope to the outside thereof. The insertion portion 4 includes a flexible outer sheath 5 and an inner sheath 6 is inserted into the outer sheath 5 so as to extend and retract along the axial direction thereof. A flexible operating wire 7 is inserted through the inner sheath 6 so as to extend and retract along the axial direction. An engaging member 8 having a hook shape is fixed to the distal end of the operating wire 7. A flexible cutting sheath 9 is fitted onto the outer periphery of the inner sheath 6 so as to extend and retract along the axial direction. A cutting member 10 as means for cutting a ligature wire to be described later is fixed to the distal end of the cutting sheath 9. The cutting member 10 has an annular shape covering the outer periphery of the inner sheath 6 and a cutting edge 11 forming an acute angle about the axial direction are formed around the distal end thereof. The cutting member 10 is made of a metal material such as stainless steel.

Here, the outer sheath 5 is made of flexible plastic such as polyethylene and PTFE (polytetrafluoroethylene) and has an inner diameter of φ2 to φ5 mm. The inner sheath 6 and the cutting sheath 9 are also made of flexible plastic such as polyethylene and PTFE, but may additionally include a metal mesh added thereto or may be made of a metal coil. The operating wire 7 is made of a twisted metal wire of stainless steel or the like.

The distal end of the insertion portion 4 having the above-mentioned configuration is provided with a handle operating section 12 for allowing an operator to perform an operation thereof outside the body. The handle operating section 12 includes a handle 13 to which the proximal end of the outer sheath 5 is fixed, a cutting operating portion 14 to which the proximal end of the cutting sheath 9 is fixed, an elongated operating section body 15 to which the proximal end of the inner sheath 6 is fixed, and a slider 16 which is attached to the operating section body 15 so as to extend and retract along the longitudinal direction. The proximal end of the operating wire 7 is fixed to the slider 16. A finger laying ring 17 is disposed at the proximal end of the operating section body 15.

The medical ligature tool 2 fitted to the distal end of the operation unit 3 includes a ligature wire 20 serving as a suture and ligature member for ligaturing a biological tissue. The ligature wire 20 is a thread made of a synthetic resin such as nylon and polyolefin, a fine metal wire of stainless steel or the like, silk, a bioabsorbable material and has a diameter of φ0.2 to φ1 mm. The ligature wire forms a loop portion 21 by folding back the wire at the center thereof in the longitudinal direction. As the ligature wire 20, a single wire can be used, but a twisted wire or a woven wire may also be used. End portions of the ligature wire 20 which form a pair by forming the loop portion 21 are press-fitted so that it penetrates a hole of a tube-shaped stopper 22. The stopper 22 is a fixing member for allowing the ligature wire 20 to extend and retract. When the stopper 22 is advanced toward the distal end with the ligature wire 20 bound, the diameter of the loop portion 21 is reduced. When the stopper is retracted, the diameter of the loop portion 21 is enlarged. The stopper 22 is made of rubber such as silicon rubber and fluorine rubber or various thermoplastic elastomers and a knot of a thread may be used instead of the tube-shaped member. A pair of end portions of the ligature wire 20 drawn out of the stopper 22 is inserted through a tube-shaped holding member 23, and one end thereof is folded back and inserted into a connecting pipe 24 as a fixing tool into which the other end of the ligature wire 20 is inserted, where both end portions are fixed with an adhesive or the like. A folding-back portion 25 on the proximal side of the ligature wire 20 is formed in a loop shape, which engages with an engaging member 8 of the operating wire 7.

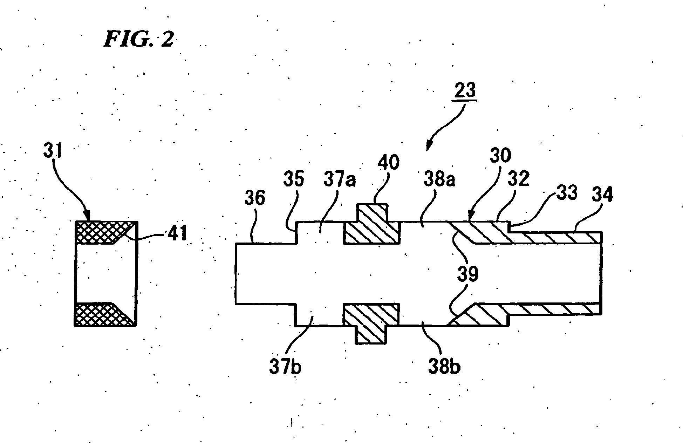

As shown in the exploded diagram of FIG. 2, the holding member 23 includes a first cylindrical member 30 on the proximal side and a second cylindrical member 31 which can fitted to the distal end portion of the first cylindrical member 30. A proximal diameter-reduced portion 34 of which the diameter is reduced by a stepped portion 33 is formed on the proximal side of a body portion 32 of the first cylindrical member 30 and a distal diameter-reduced portion 36 of which the diameter is reduced by a stepped portion 35 is formed on the distal end of the body portion 32. A pair of slits 37a and 37b is formed to extend over the stepped portion 35 along the longitudinal direction from the distal diameter-reduced portion 36. The slits 37a and 37b are formed so as to penetrate from the outer periphery to the inner periphery on one diameter of the first cylindrical member 30. In the portion closer to the proximal end of the slits 37a and 37b, proximal lateral holes 38a and 38b are formed in the same line as the slits 37a and 37b along the longitudinal direction of the first cylindrical member 30. The proximal lateral holes 38a and 38b are formed so as to penetrate from the outer periphery to the inner periphery of the first cylindrical member 30. The proximal lateral holes 38a and 38b have a slope 39 which gradually expands the opening length to the proximal side from the outer peripheral opening to the inner peripheral opening. An annular protrusion 40 formed by allowing the body portion 32 to protrude outwardly in the radial direction is formed between the slits 37a and 37b and the proximal lateral holes 38a and 38b in the axial direction of the first cylindrical member 30. A contact surface 40a is formed on the proximal side of the annular protrusion 40 and the contact surface 40a is substantially perpendicular to the axial direction of the first cylindrical member 30.

The second cylindrical member 31 has an inner diameter which can be fitted to the outer periphery of the distal diameter-reduced portion 36 of the first cylindrical member 30. The inner diameter of the second cylindrical member 31 is preferably larger than twice of the diameter of the ligature wire 20 and is set to such a size to easily release the fitting to the first cylindrical member 30. A tapered surface 41 which increases the diameter toward the proximal opening surface is formed in the inner periphery of the proximal end of the second cylindrical member 31. The length in the axial direction of the second cylindrical member 31 is preferably as short as possible.

As shown in FIG. 1, in a state where the second cylindrical member 31 is fitted to the first cylindrical member 30, a pair of distal lateral holes 42a and 42b through which the ligature wire 20 can be inserted is formed by the tapered surface 41 on the proximal side of the second cylindrical member 31 and the slits 37a and 37b as the lateral hole forming portions. The pair of ligature wires 20 is drawn out of the stopper 22, is guided to the second cylindrical member 31 from the distal opening of the holding member 23, is inserted through the pair of distal lateral holes 42a and 42b, respectively, drawn out to the outer peripheral surface of the first cylindrical member 30, extends over the annular protrusion 40, is guided into the first cylindrical member 30 through the proximal lateral holes 38a and 38b formed along the axial direction, and is drawn out of the proximal opening. In this way, a holding portion for holding the ligature wire 20 is formed in the way from the distal lateral holes 42a and 42b to the proximal lateral holes 38a and 38b. The outer diameter of the proximal diameter-reduced portion 34 of the holding member 23 is smaller than the inner diameter of the inner sheath 6 and the outer diameter of the holding member 23 other than the proximal diameter-reduced portion 34 and the annular protrusion 40 is substantially equal to the outer diameter of the inner sheath 6. The holding member 23 is made of a metal material such as stainless steel or plastic such as po ypropylene, ABS (acrylonitrile-butadiene resin), or polycarbonate.

Next, operations of this example will be described.

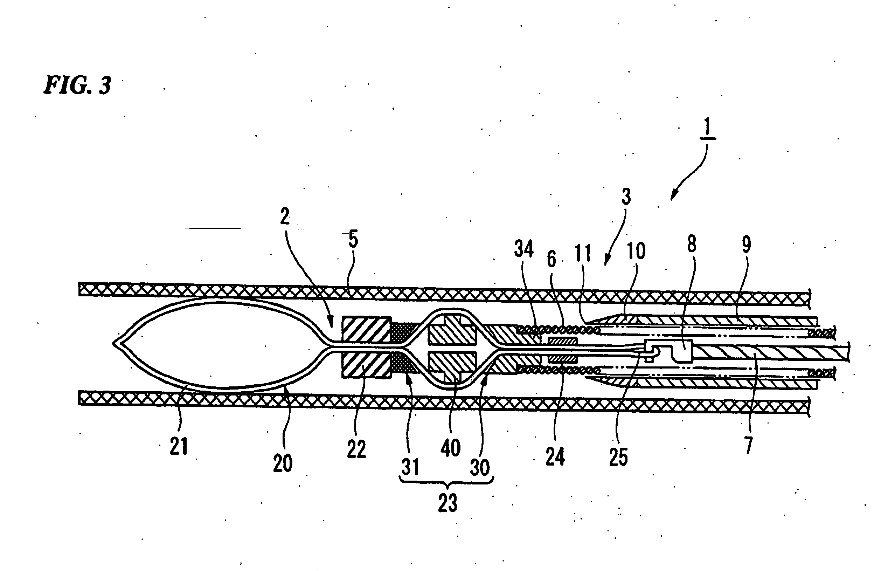

First, the medical ligature tool 2 is mounted on the operation unit 3. Specifically, the operating wire 7 is advanced by operating the slider 16 and the engaging member 8 on the distal end of the operating wire 7 is protruded from the distal openings of the outer sheath 5 and the inner sheath 6. In this state, when the folding-back portion 25 of the ligature wire 20 is hooked to the engaging member 8 and locked, the operating wire 7 is retracted by drawing back the slider 16. Accordingly, the engaging member 8 is drawn into the inner sheath 6 and the proximal diameter-reduced portion 34 of the holding member 23 is inserted into and supported by the inner sheath 6. Thereafter, the outer sheath 5 is advanced by operating the handle 13. Accordingly, as shown in FIG. 3, the ligature wire 20 is received in the outer sheath 5 and the loop portion 21 contracts.

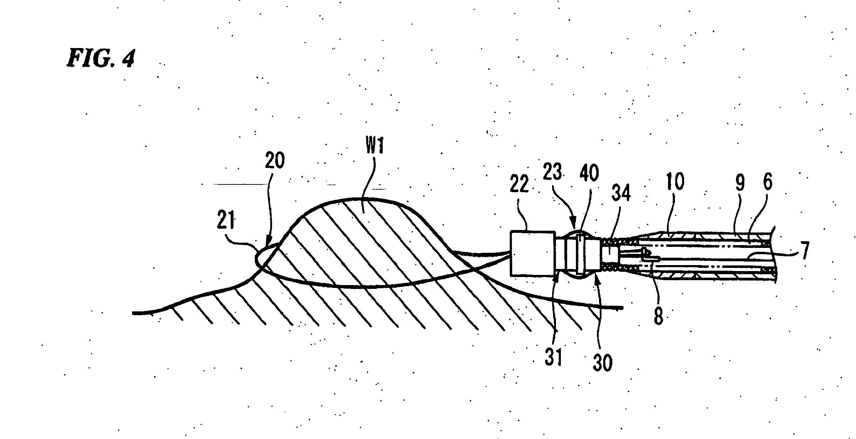

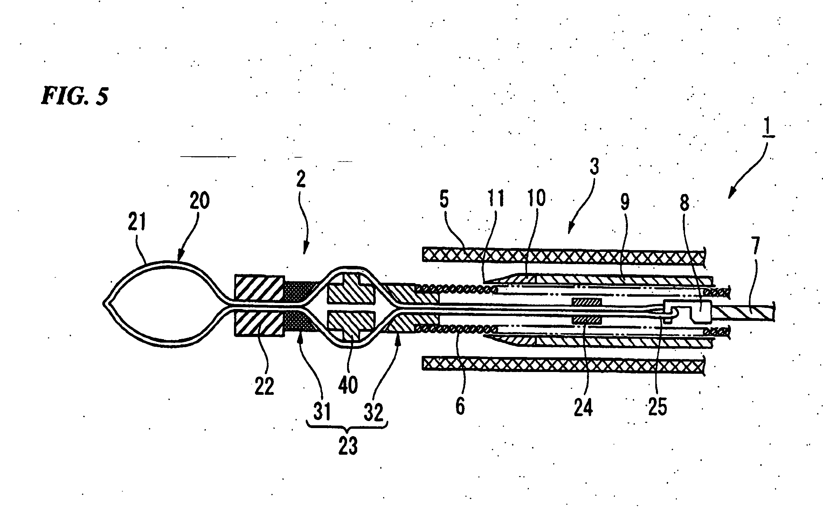

When the medical ligature tool 2 is mounted, the insertion portion 4 of the operation unit 3 is inserted through the channel of the endoscope and is inserted into the body along with the endoscope. When the distal end of the insertion portion 4 is guided to a target site in the body, the outer sheath 5 is retracted by operating the handle 13 while observing a pathological lesion portion such as polyps with the endoscope. As shown in FIG. 1, the loop portion 21 of the ligature wire 20 protrudes from the distal opening of the outer sheath 5 and is restored by means of the elastic force thereof to enlarge the diameter. Next, as shown in FIG. 4, the loop portion 21 of the ligature wire 20 is hooked to a root of a pathological lesion portion W1 while observing the pathological portion with the endoscope. In this state, when the operating wire 7 is retracted by operating the slider 16, the proximal end of the ligature wire 20 is drawn into the inner sheath 6 via the engaging portion 8 as shown in FIG. 5. Accordingly, the stopper 22 and the holding member 23 are relatively moved toward the distal end of the ligature wire 20.

As a result, the diameter of the loop portion 21 of the ligature wire 20 is reduced and the pathological lesion portion W1 is tightly fastened. In this way, the pathological lesion portion is ligatured with the ligature wire 20, thereby stopping the blood circulation to the pathological lesion portion W 1.

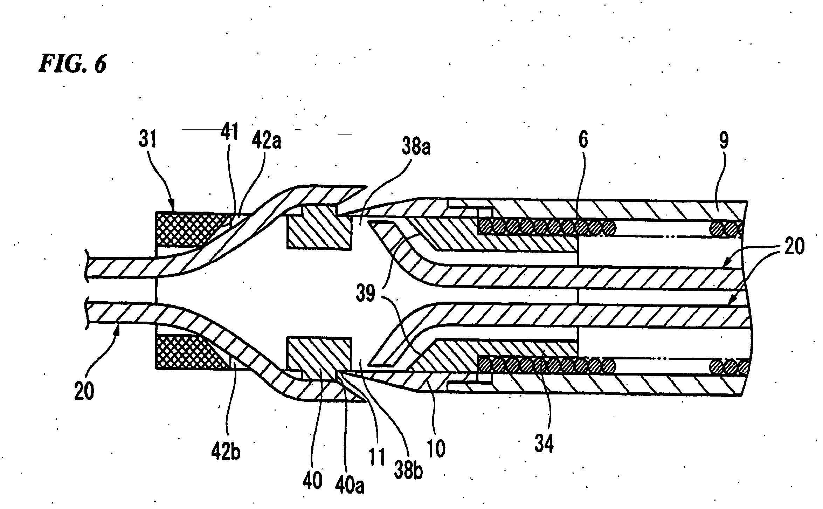

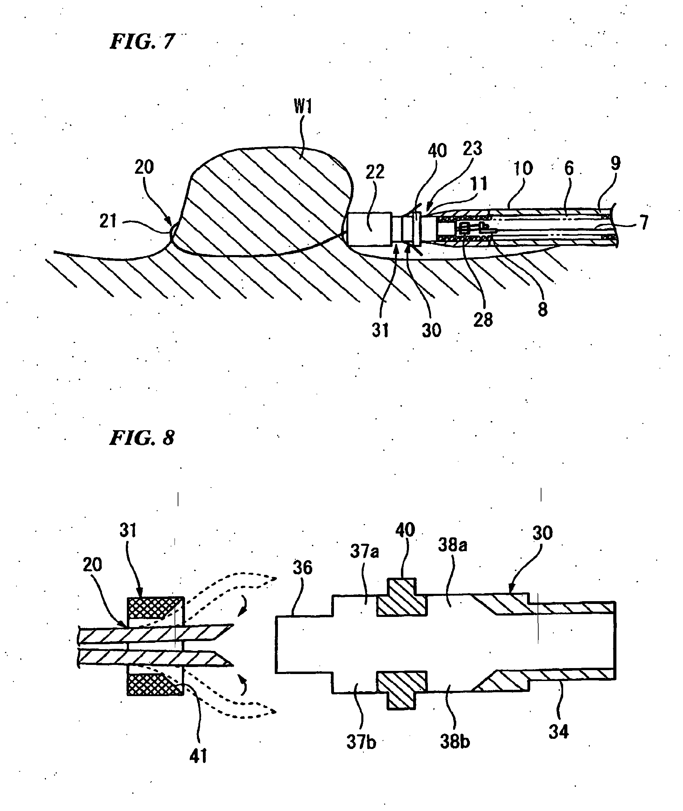

When the cutting sheath 9 is extended by operating the cutting operating section 14 with the slider 16 kept in place, the cutting sheath 9 is guided by the inner sheath 6 so as to extend. Accordingly, the cutting member 10 disposed on the distal end thereof is extended while being fitted to the proximal end of the first cylindrical member 30 of the holding member 23. As a result, as shown in FIGS. 6 and 7, the cutting edge 11 of the cutting member 10 rubs and cuts the ligature wire 20 held by the holding portion located in the path thereof, that is, the ligature wire 20 exposed from the proximal lateral holes 38a and 38b of the first cylindrical member 30. At this time, since the proximal end portion of the ligature wire 20 is suspended by the operating wire 7 and a tension is applied thereto, the ligature wire can be easily cut by the cutting edge 11. The ligature wires 20 passing through the proximal lateral holes 38a and 38b are not cut simultaneously, but may be cut individually. However, since the ligature wires 20 are coupled to the connecting pipe 24, the tension is maintained even when only one end is cut. Accordingly, the cutting effect by the cutting edge 11 is not varied.The cutting sheath 9 can be advanced until the cutting edge 11 comes in contact with the contact surface 40a of the annular protrusion 40

When the ligature wire 20 is cut in the vicinity of the proximal lateral holes 38a and 38b, the force for drawing the holding member 23 to the inner sheath 6 disappears and thus the holding member 23 can be separated from the inner sheath 6. By cutting the ligature wire 20 passing through the outer periphery of the holding member 23, the portion of the medical ligature tool 2 detained in the body is separated from the operation unit 3. Here, the force for restoring the ligature wire 20 to a straight shape acts on the uncut ligature wire 20 by being curved outward to allow the ligature wire to pass through the outer periphery of the holding member 23. By cutting the portion curved outward, the force for restoring the ligature wire to the straight shape acts so that the cut end of the ligature wire 20 is inserted inward from the distal lateral holes 42a and 42b and the proximal lateral holes 38a and 38b and passes through them. Accordingly, when the cutting sheath 9 is retracted by operating the cutting operating section 14 and then the insertion portion 4 of the operation unit 3 is pulled out of the channel of the endoscope, the cut end of the ligature wire 20 passes through the distal lateral holes 42a and 42b and the proximal lateral holes 38a and 38b causing the first cylindrical member 30 falling off from the ligature wire 20. As a result, as shown in FIG. 8, the first cylindrical member 30 falls off from the ligature wire 20. At this time, since the first cylindrical member 30 is loosely supported by the inner sheath 6, the first cylindrical member 30 is easily separated from the operation unit 3. The end portion of the ligature wire 20 locked to the engaging member 8 is pulled out of the body along with the operation unit 3.

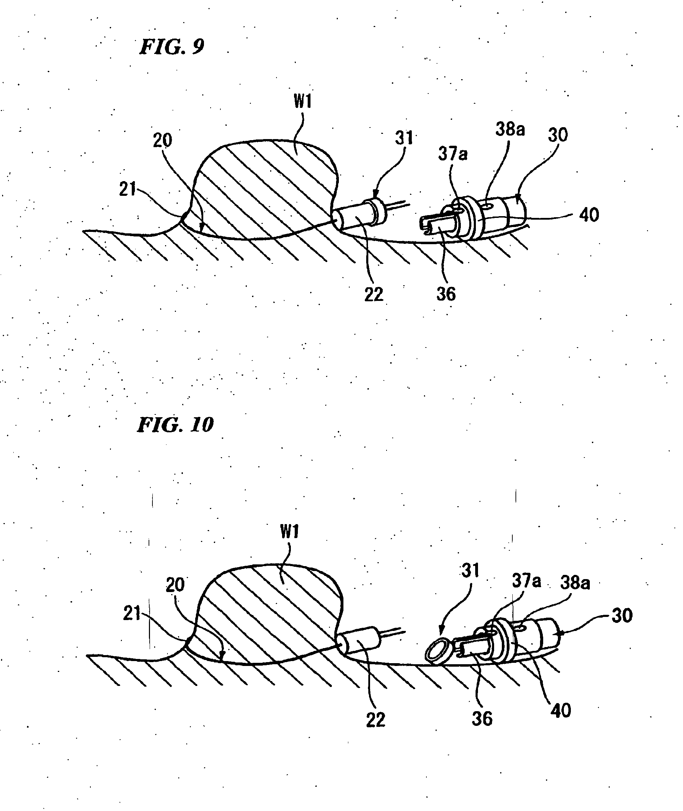

Since the end portion of the ligature wire 20 close to the second cylindrical member 31 is stretched in a straight shape by means of the separation of the first cylindrical member 30, the second cylindrical member 31 also separates from the end portion. Accordingly, as shown in FIG. 10, the ligature wire 20 tightly fastened the pathological lesion portion W1 by the stopper 22 is detained in the body, whereby the procedure of tightly fastening the pathological lesion portion W1 is completed. The first cylindrical member 30 and the second cylindrical member 31 separated from the ligature wire 20 are spontaneously discharged from the body through a digestive canal.

According to this example, the holding member 23 includes the first cylindrical member 30 and the second cylindrical member 31 and the first cylindrical member 30 and the second cylindrical member 31 can be separated before and after the distal lateral holes 42a and 42b through which the ligature wire 20 is inserted. Accordingly, when the ligature wire 20 is cut by the cutting member 10, the cut end of the ligature wire 20 can be rapidly separated from the first cylindrical member 30 and thus the portion detained in the body can be reliably separated from the first cylindrical member 30. Since the second cylindrical member 31 has a cylindrical shape of which the length in the axial direction is small, the second cylindrical member 31 can be easily separated from the cut end of the ligature wire 20. Accordingly, since the holding member 23 can be rapidly and reliably separated from the ligature wire 20 detained in the body, it is not necessary to insert<a particular endoscopic treatment tool through the endoscope for the separation; thereby operation time can be reduced.

The holding member 23 may have a constant outer diameter from the distal lateral holes 42a and 42b to the proximal lateral holes 38a and 38b, without having the annular protrusion 40.

### Second example

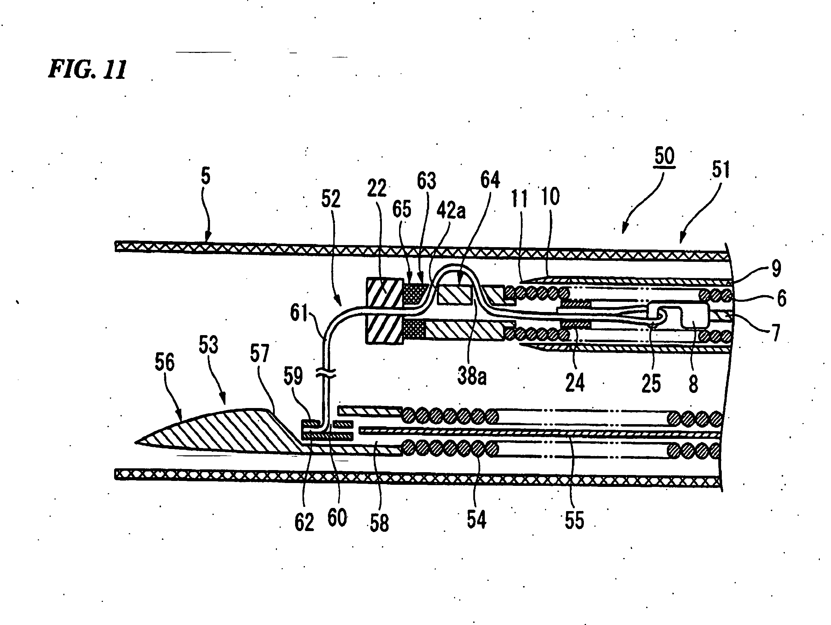

A second example will be described with reference to FIGS. 11 to 17. The same elements as the first example are denoted by the same reference numerals and repeated descriptions are omitted.

A medical suture and ligature instrument according to this example is a medical suture instrument for suturing a pathological lesion portion to stop bleeding and the like. That is, as shown in FIG. 11, the medical suture instrument 50 includes an operation unit 51 and a medical suture tool (medical suture and ligature tool) 52 mounted on the operation unit 51. In the operation unit 51, an inner sheath 6 and a medical puncture unit 53 having a suture needle as a tissue puncture member are disposed in an outer sheath 5 so as to independently extend and retract. A cutting sheath 9 is disposed on the outer periphery of the inner sheath 6 and an operating wire 7 is inserted through the inner sheath 6.

The medical puncture unit 53 has a flexible puncture portion body 54 formed by winding a metal such as stainless steel in a close coiling shape forming into a pipe shape. The puncture portion body 54 extends along the outer sheath 5 and a flexible extrusion rod 55 is inserted therein so as to extend and retract. The proximal ends of the puncture portion body 54 and the extrusion rod 55 are connected to a handle operating section not shown and can be operated to extend and retract. A needle portion 56 is attached to the distal end of the puncture portion body 54. The needle portion 56 has an acute distal end and extends along the axial line of the puncture portion body 54, and the outer diameter thereof is equal to the outer diameter of the puncture portion body 54. The needle portion 56 is made of a metal material such as stainless steel and an opening 57 is formed in the side surface thereof. A reception portion 58 communicating with the hollow of the puncture portion body 54 is formed in the opening 57 and a separation-preventing tip 59 of the medical suture tool 52 is received therein.

The medical suture tool 52 has a separation-preventing tip 59 as an engaging member and the separation-preventing tip 59 has a pipe shape in which a slit 60 is formed in the longitudinal direction. The slit 60 extends to the substantial center in the longitudinal direction of the separation-preventing tip 59 and a suture thread 61 is inserted therefrom. The suture thread 61 is fixed to the separation-preventing tip 59 by the use of a caulking portion 62 formed to caulk the end portion of the separation-preventing tip 59 and extends by a predetermined length therefrom. The end portion thereof is folded back after passing through a stopper 22 and a holding member 63 so as to form a folding-back portion 25 and is connected to a connecting pipe 24. In the medical suture tool 52, the separation-preventing tip 59 is housed in the receiving portion 58 of the needle portion 56 so as to be substantially parallel to the axial line of the puncture portion body 54 and the folding-back portion 25 of the suture thread 61 is locked to the engaging member 8 of the operating wire 7. Here, the holding member 63 includes a first cylindrical member 64 and a second cylindrical member 65 and has the same configuration as the holding member 38a of the first example except that only one distal lateral hole 42a and one proximal lateral hole 38a are formed.

Next, operations of this example will be described.

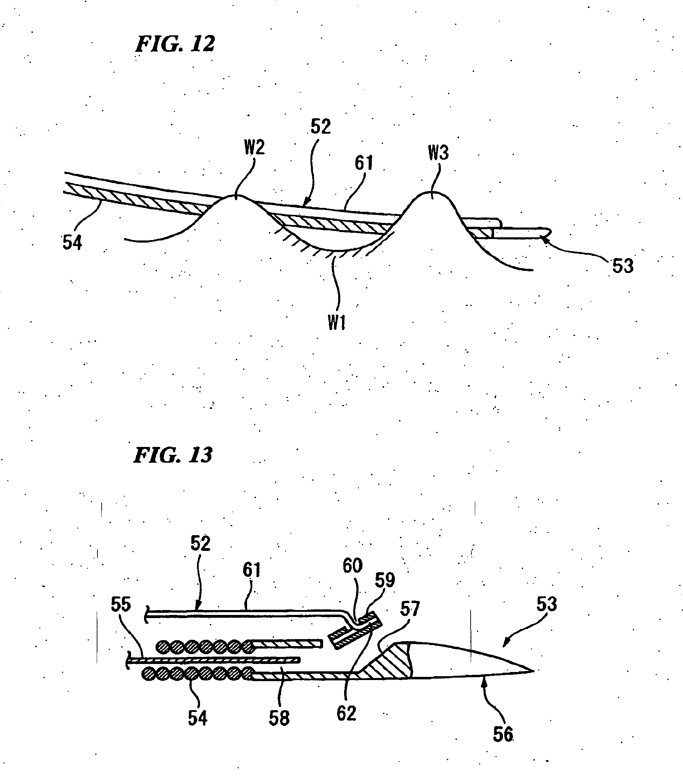

First, the distal end of an endoscope is introduced into the vicinity of a pathological lesion portion to be sutured while observing an image of the endoscope and the medical suture instrument 50 is inserted into a channel of the endoscope. At this time, the inner sheath 6 and the medical puncture unit 53 are housed in the outer sheath 5. When it is observed that the outer sheath 5 protrudes from the distal end of the endoscope, the outer sheath 5 is retracted to expose the medical puncture unit 53. Then, By operating the medical puncture unit 53, as shown in FIG. 12, a biological tissue W2 before the pathological lesion portion W1 required to stop the bleeding thereof and an opposite biological tissue W3 with the pathological lesion portion W1 interposed therebetween are sequentially punctured with the needle portion 56. Accordingly, the suture thread 61 penetrates the biological tissues W2 and W3 sequentially along with the needle portion 56.

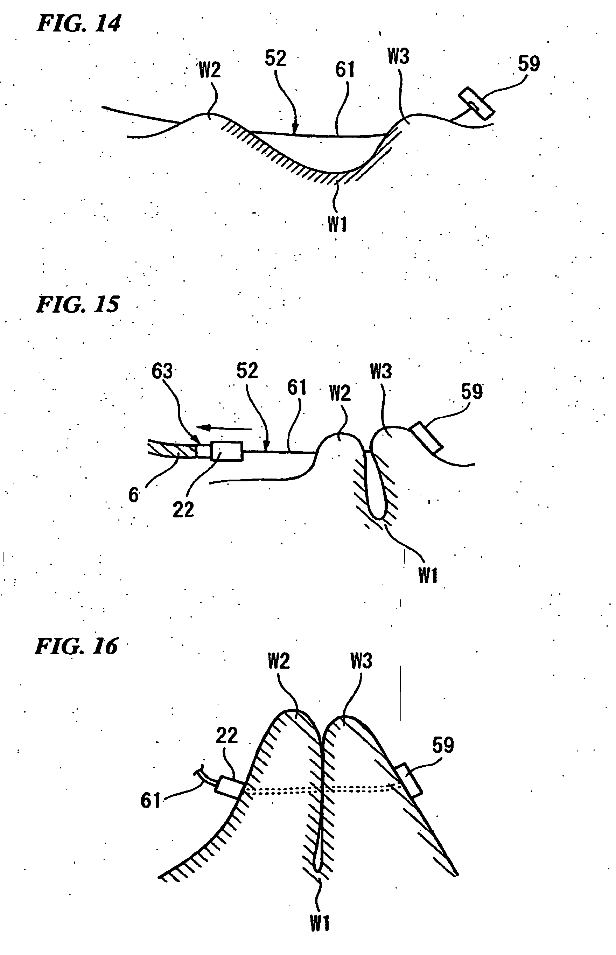

In this state, as shown in FIG. 13, the extrusion rod 55 is advanced and the separation-preventing tip 59 is extruded from the receiving portion 58. Accordingly, the separation-preventing tip 59 is separated from the medical puncture unit 53. Thereafter, when the puncture portion body 54 is retracted, the needle portion 56 is pulled out from the biological tissues W2 and W3. However, as shown in FIG. 14, the separation-preventing tip 59 remains fixed to the biological tissue W3 and the suture thread 61 maintains the state where it penetrates the biological tissues W2 and W3. Then, when the biological tissues W2 and W3 are drawn inward while relatively extruding the stopper 22 by drawing back the operating wire 7, the pathological lesion portion W1 as the bleeding portion is closed and the biological tissues W2 and W3 are sutured as shown in FIG. 15. Thereafter, when the cutting sheath 9 is pushed inward to enter between the proximal lateral hole 38a and the cutting member 10, the proximal side of the suture thread 61 is cut.

When the proximal side of the suture thread 61 is cut, the inner sheath 6 and the holding member 63 are separated from each other similarly to the first example. In the medical suture tool 52, a portion where the biological tissues W2 and W3 are sutured is separated from the operation unit 51. In this state, when the endoscope is pulled out, the suture thread 61 is disengaged from the first cylindrical member 64 by the force for restoring the cut end of the suture thread 61 suturing the biological tissues W2 and W3 from the curved state with the first cylindrical member 64 to the straight shape and the first cylindrical member 64 falls off. In this way, the second cylindrical member 65 is separated from the end portion of the suture thread 61 changed to the straight shape and the first and second cylindrical members 64 and 65 are discharged from the body through a digestive tract. As shown in FIG. 16, the separation-preventing tip 59, the stopper 22, and the suture thread 61 penetrating the biological tissues W2 and W3 are detained in the body and thus the state where the biological tissues W2 and W3 are sutured is maintained.

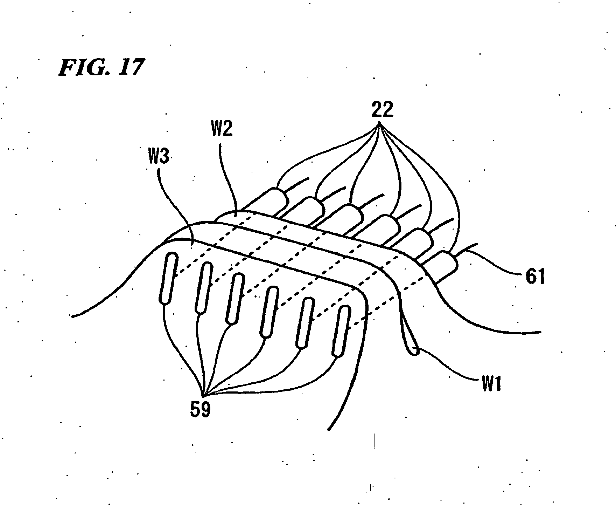

According to this example it is possible to suture the biological tissues W2 and W3 by allowing the suture thread 61 to penetrate the biological tissues and it is also possible to reliably separate the portion suturing the biological tissues W2 and W3 and the holding member 63 in the medical suture tool 52 from each other. Other advantages are same as those of the first example. Here, since the holding member 63 is rapidly and reliably separated, it is possible to suture the biological tissues W2 and W3 by the use of a plurality of suture threads 61. For example, as shown in FIG. 17, it is possible to suture the biological tissues W2 and W3 over a wide range by the use of the plurality of suture threads 61. In this case, since the holding member 63 is reliably separated from one medical suture tool 52 and then a next suture can be performed, the gaps between the neighboring suture threads 61 can be reduced, thereby more reliably suturing the biological tissues W2 and W3.

### Third example

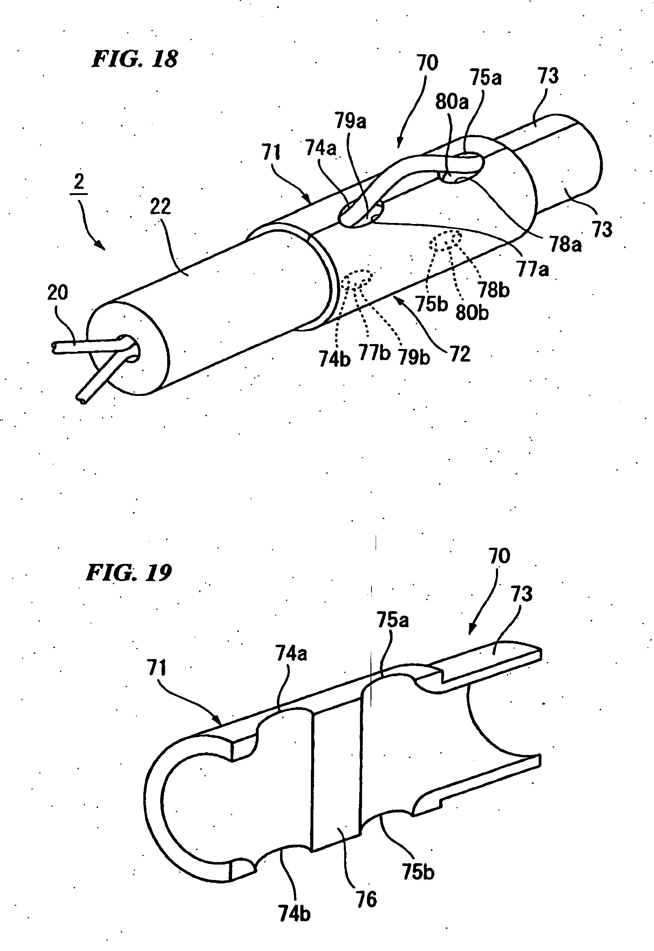

A third example will be described mainly with reference to FIGS. 18 and 19. The same elements as the first example are denoted by the same reference numerals and repeated descriptions are omitted.

A medical ligature tool according to this example is different from that of the first example in the configuration of the holding member. As shown in FIG. 18, a holding member 70 includes a first member 71 and a second member 72 which are formed by dividing a cylindrical member into two members in the longitudinal direction. As shown in FIGS. 18 and 19, in the first member 71, a proximal diameter-reduced portion 73 is disposed on the proximal side and a pair of cut portions 74a and 74b and a pair of cut portions 75a and 75b are formed between the distal end and the proximal diameter-reduced portion 73 so as to make concave a part of a fitting surface to the second member 72. The distal cut portions 74a and 74b are disposed coaxially with the axial line perpendicular to the longitudinal direction and the proximal cut portions 75a and 75b are disposed in the same way. On the inner periphery of the first member 71, a reinforcing rib 76 is disposed between the pair of cut portions 74a and 74b and the pair of cut portions 75a and 75b so as to be perpendicular to the longitudinal direction. The rib 76 is flush with the fitting surface to the second member 72. On the other hand, the second member 72 is symmetric to the first member 71. That is, a pair of cut portions 77a and 77b and a pair of cut portions 78a and 78b are formed at positions opposed to the pair of cut portions 74a and 74b and the pair of cut portions 75a and 75b of the first member 71.

By coupling the first member 71 and the second member 72, a distal lateral hole 79a is formed of the cut portions 74a and 77a and a distal lateral hole 79b is formed of the cut portions 74b and 77b. A proximal lateral hole 80a is formed of the cut portions 75a and 78a and a proximal lateral hole 80b is formed of the cut portions 75b and 78b. That is, the cut portions 74a, 74b, 75a, 75b, 77a, 77b, 78a, and 78b are lateral hole forming portions for forming the lateral holes 79a, 79b, 80a, and 80b by coupling the first and second members 71 and 72. A holding portion for holding the ligature wire 20 is formed between the distal lateral hole 79a and 79b to the proximal lateral hole 80a and 80b. The maximum outer diameter of the holding member 70 is substantially equal to the inner diameter of the cutting member 10 (see FIG. 1) and the proximal diameter-reduced portion 73 has a size sufficient to engage with the inside of the inner sheath 6. By allowing the holding member to engage with the inner sheath 6, a coupled state of the holding member 70 is maintained

Next, operations of this example will be described.

When the proximal end portion of the ligature wire 20 is cut by the cutting member 10 after ligaturing a pathological lesion portion W1, the holding member 70 is separated from the inner sheath 6 and the portion of the medical ligature tool 2 detained in the body is separated from the operation unit 3. When the insertion portion 4 is pulled out of the channel of the endoscope, the proximal diameter-reduced portion 73 of the holding member 70 is disengaged from the inner sheath 6, the force of coupling the first and second members 71 and 72 disappears, and the first member 71 and the second member 72 are separated from each other and are individually separated from the ligature wire 20. The separated first and second members 71 and 72 are spontaneously discharged from the body through a digestive tract.

According to this example, since the holding member 70 includes the first and second members 71 and 72 which can be separated from each other in a direction perpendicular to the axial direction, the direction of the frictional force resulting from the contact between the ligature wire 20 and the holding member 70 is different from the direction in which the holding member 70 is divided by about 90 degrees, thereby reliably separating the holding member 70 from the ligature wire 20. In addition, since the shape of the holding member 70 is maintained by supporting the first and second members 71 and 72 to the inner sheath 6, the coupling of the first and second members 71 and 72 are easily released at the time of drawing back the inner sheath 6, thereby reliably separating the holding member 70 from the ligature wire 20.

### Fourth example

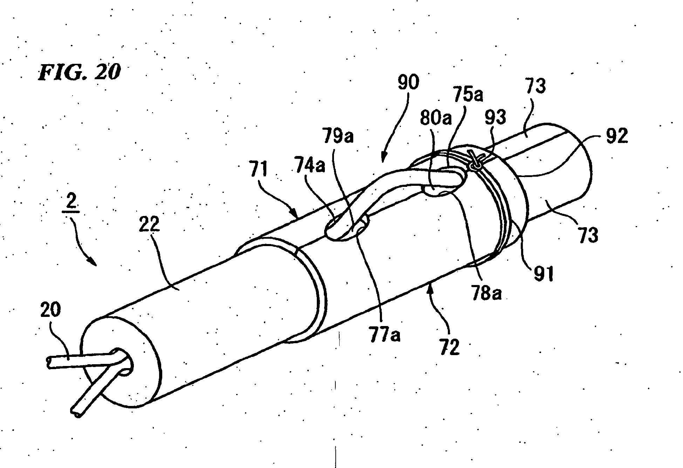

A fourth example will be described mainly with reference to FIG. 20. The same elements as the first example are denoted by the same reference numerals and repeated descriptions are omitted.

A medical ligature tool according to this example is different from the third example in the configuration of the holding member. As shown in FIG. 20, a holding member 90 includes a first member 71, a second member 72, and a coupling wire 91 for tightly binding the first and second members 71 and 72. The coupling wire 91 is wound on the outer peripheries of the first and second members 71 and 72 between a stepped portion 92 forming a proximal diameter-reduced portion 73 and proximal lateral holes 80a and 80b (see FIG. 18) and couples the first and second members 71 and 72 with a knot 93. The knot 93 is disposed in a line extending in the longitudinal direction from the forming position of the proximal lateral hole 80a and is located outside the outer periphery of the cutting edge 11 of the cutting member 10. The coupling wire 91 is made of a thread of a synthetic resin such as nylon (polyamide resin) and polypropylene, a silk thread, and a thread of a bioabsorbable material and the diameter thereof is in the range of φ0.2 to φ1 mm. The coupling wire 91 may be a single wire or may be a twisted wire or a woven wire.

Next, operations of this example will be described.

When the cutting member 10 is advanced after ligaturing a pathological lesion portion W1, the cutting edge 11 cuts off the knot 93 of the coupling wire 91. Thereafter, the cutting member 10 is further advanced to cut the ligature wire 20. When the insertion portion 4 is retracted, the inner sheath 6 and the holding member 90 are separated from each other, and the portion detained in the body in the medical ligature tool 2 is separated from the operation unit 3. When the operation unit 3 is pulled out, the proximal diameter-reduced portion 73 of the holding member 90 is separated from the inner sheath 6. At this time, since the coupling wire 91 is cut in advance, the force acting to couple the first and second members 71 and 72 disappears and the first member 71 and the second member 72 are separated from each other and separated from the ligature wire 20. The separated first and second members 71 and 72 and the cut coupling wire 91 are spontaneously discharged from the body through a digestive tract.

According to this example, since the first member 71 and the second member 72 are coupled to each other with the coupling wire 91, it is possible to maintain the shape of the holding member 90 even when it does not engage the inner sheath 6. Accordingly, it is easy to conserve the medical ligature tool 2 and it is also easy to mount the medical ligature tool on the operation unit 3. The advantage of the ligaturing is equal to that of the third example.

### Embodiment of the invention

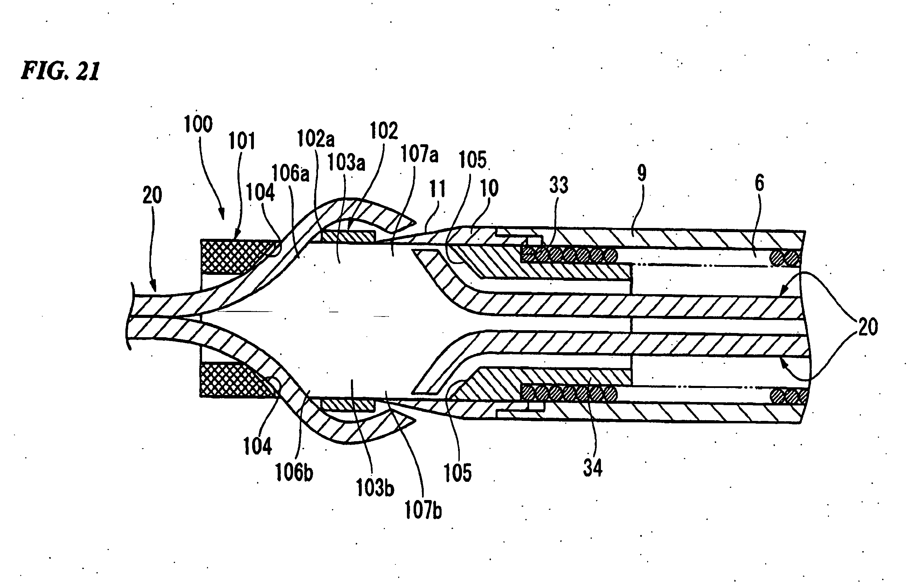

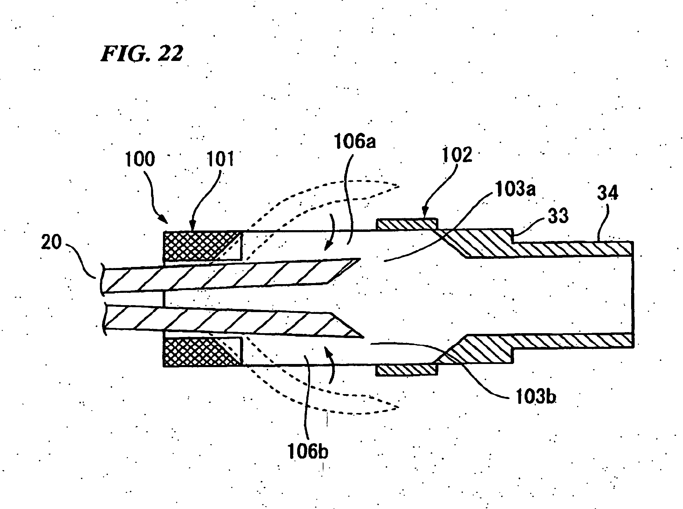

An embodiment of the invention will be described mainly with reference to FIGS. 21 and 22. The same elements as the first example are denoted by the same reference numerals and repeated descriptions are omitted.

A medical ligature tool according to this embodiment is different from that of the first example in the configuration of the holding member. As shown in FIG. 21, a holding member 100 includes a first cylindrical member 101 which is long and thin and a second cylindrical member 102 mounted to cover the outer periphery of the first cylindrical member 101. In the first cylindrical member 101, a proximal diameter-reduced portion 34 is formed on the proximal side and a pair of elongated lateral holes 103a and 103b penetrating the outer periphery and the inner periphery of the first cylindrical member 101 is disposed between a stepped portion 33 of a proximal diameter-reduced portion 34 and the distal end surface so as to extend in the axial direction. The lateral holes 103a and 103b are lateral hole forming portions disposed in one diameter of the first cylindrical member 101 and the lateral holes 103a and 103b have tapered surfaces 104 and 105, respectively, so as to increase the length of the opening in the longitudinal direction from the outer periphery to the inner periphery. The second cylindrical member 102 is mounted to cover a portion of the lateral holes 103a and 103b. The second cylindrical member 102 is slidable in the longitudinal direction of the first cylindrical member 101 and the length in the longitudinal direction is smaller than the opening length on the outer periphery side of the lateral holes 103a and 103b. Accordingly, in the initial state, a pair of distal lateral holes 106a and 106b penetrating the first cylindrical member 101 on the distal side and a pair of lateral holes 107a and 107b penetrating the first cylindrical member 101 on the proximal side are formed by the second cylindrical member 102 and the lateral holes 103a and 103b. In the holding member 100, a holding portion for holding the ligature wire 20 is formed between the distal lateral holes 106a and 106b and the proximal lateral holes 107a and 107b.

Operations of this embodiment will be described.

When the cutting member is advanced after ligaturing a pathological lesion portion W1, the cutting edge 11 advanced to cut the ligature wire 20. When the insertion portion 4 is retracted, the inner sheath 6 is separated from the holding member 100 and the portion of the medical ligature tool 2 detained in the body and the operation unit 3 are separated from each other. When the operation unit 3 is pulled out, the cut ends of the ligature wire 20 widened by the distal lateral holes 106a and 106b come close to each other. The force generated at that time acts on a contact portion 102a with the second cylindrical member 102 and the second cylindrical member 102 is made to slide to the proximal side by this force. As a result, as shown in FIG. 22, the length in the longitudinal direction of the distal lateral holes 106a and 106b increases. By expanding the distal lateral holes 106a and 106b, the end of the ligature wire 20 is restored to the straight shape. Accordingly, the first cylindrical member 101 is separated from the ligature wire 20, only the ligature wire 20 tightly bound by a stopper 22 (see FIG. 1) is detained in the body, and the separated first and second cylindrical members 101 and 102 are spontaneously discharged from the body through a digestive tract.

According to this embodiment, the holding member 100 is configured by fitting the second cylindrical member to the first cylindrical member 101 so as to be slidable and the lengths of the distal lateral holes 106a and 106b through which the ligature wire 20 is inserted can be adjusted by the sliding amount of the second cylindrical member 102. Accordingly, after cutting the ligature wire 20, the ligature wire 20 can be made to easily come off by increasing the lengths of the distal lateral holes 106a and 106b, thereby reliably separating the holding member 100 from the ligature wire 20.

The invention is not limited to the above-mentioned embodiment, but may be applied widely.

For example, in the embodiment, the separation-preventing tip 59 and the suture thread 61 of the second example may be used instead of the ligature wire 20. Also, the annular protrusion 40 may be provided.

The holding member is not limited to the of the above-mentioned embodiment, but may have any configuration as long as the distal lateral hole and the suture thread 61 are disengaged from each other by enlarging or opening the distal lateral hole.

## Claims

1. A medical suture and ligature tool (2) comprising:
a flexible suture and ligature member (20) adapted to suture or ligature a biological tissue; and ,
a holding member (100) having a holding portion adapted to hold the suture and ligature member (20) in a path along which a cutting member (10) adapted to cut the suture and ligature member (20) moves,
**characterized in that**
the holding member (100) further has:
a first member (101) formed in a cylindrical shape, wherein at least one lateral hole (103a, 103b) is formed on the first member (101); and
a second member (102) formed in a cylindrical shape, the second member (102) being mounted on the first member (101) so as to cover an outer periphery of the first member (101) and to be slidable on the outer periphery of the first member (101) in a longitudinal direction of the first member (101),
wherein a length of the second member (102) in the longitudinal direction is shorter than a length of an opening of the lateral hole (103a, 103b) in the longitudinal direction, and
wherein the second member (102) is disposed on a middle portion of the lateral hole (103a, 103b) in the longitudinal direction, and the suture and ligature member (20) is inserted through both a portion of the lateral hole (103a, 103b) closer to a distal side of the first member (101) than the second member (102) and a portion of the lateral hole (103a, 103b) closer to a proximal side of the first member (101) than the second member (102).

2. The medical suture and ligature tool (2) according to Claim 1, wherein
the first member (101) has a tapered surface (104, 105) facing the lateral hole (103a, 103b), and
the tapered surface (104, 105) is formed so as to increase the length of the opening of the lateral hole (103a, 103b) in the longitudinal direction from the outer periphery of the first member (101) to an inner periphery of the first member (101).

3. A medical suture and ligature instrument (1) comprising:
the medical suture and ligature tool (2) according to Claim 1 or 2; and
an operation unit (3), wherein the medical suture and ligature tool (2) is detachably attached to a distal end of the operation unit (3), wherein
the operation unit (3) includes:
an insertion section (4) adapted to be inserted into a body, the insertion section (4) having a distal end, a proximal end, and an elongated axis; and
the cutting member (10) provided so as to be movable relative to the insertion section (4), the cutting member having a cutting edge.

## Patentansprüche

1. Medizinisches Näh- und Ligaturgerät (2), das umfasst:
ein flexibles Näh- und Ligaturelement (20), das dazu eingerichtet ist, ein biologisches Gewebe zu nähen oder abzubinden; und
ein Halteelement (100), das einen Halteabschnitt hat, der dazu eingerichtet ist, das flexible Näh- und Ligaturelement (20) in einem Pfad zu halten, entlang dem sich ein Schneideelement (10) bewegt, das dazu eingerichtet ist, das Näh- und Ligaturelement (20) zu schneiden,
**dadurch gekennzeichnet, dass**
das Halteelement (100) ferner umfasst:
ein erstes Element (101), das in einer zylindrischen Form ausgebildet ist, wobei mindestens eine laterale Öffnung (103a, 103b) an dem ersten Element (101) ausgebildet ist; und
ein zweites Element (102), das in einer zylindrischen Form ausgebildet ist, wobei das zweite Element (102) so an dem ersten Element (101) angebracht ist, dass es einen Außenumfang des ersten Elements (101) abdeckt und an dem Außenumfang des ersten Elements (101) in einer longitudinalen Richtung des ersten Elements (101) verschiebbar ist,
wobei eine Länge des zweiten Elements (102) in der longitudinalen Richtung kürzer ist als eine Länge einer Öffnung der lateralen Öffnung (103a, 103b) in der longitudinalen Richtung, und
wobei das zweite Element (102) an einem mittleren Abschnitt der lateralen Öffnung (103a, 103b) in der longitudinalen Richtung angebracht ist und das Näh- und Ligaturelement (20) sowohl durch einen Abschnitt der lateralen Öffnung (103a, 103b), der näher an einer distalen Seite des ersten Elements (101) ist als das zweite Element (102), als auch durch einen Abschnitt der lateralen Öffnung (103a, 103b), der näher zu einer proximalen Seite des ersten Elements (101) als das zweite Element (102) ist, eingeführt ist.

2. Medizinisches Näh- und Ligaturgerät (2) gemäß Anspruch 1, wobei
das erste Element (101) eine geneigte Oberfläche (104, 105) hat, die der lateralen Öffnung (103a, 103b) zugewandt ist, und
die geneigte Oberfläche (104, 105), so geformt ist, dass sie die Länge der Öffnung der lateralen Öffnung (103a, 103b) in der longitudinalen Richtung von dem Außenumfang des ersten Elements (101) zu einem Innenumfang des ersten Elements (101) vergrößert.

3. Medizinisches Näh- und Ligaturinstrument (1), das umfasst:
das medizinische Näh- und Ligaturgerät (2) gemäß Anspruch 1 oder 2; und
eine Bedieneinheit (3), wobei das medizinische Näh- und Ligaturgerät (2) lösbar mit dem distalen Ende der Bedieneinheit (3) verbunden ist, wobei
die Bedieneinheit (3) umfasst:
einen Einführabschnitt (4), der dazu eingerichtet ist, in einen Körper eingeführt zu werden, wobei der Einführabschnitt (4) ein distales Ende, ein proximales Ende und eine langgestreckte Achse hat; und
das Schneideelement (10), das so vorgesehen ist, dass es relativ zu dem Einführabschnitt (4) beweglich ist, wobei das Schneideelement (10) eine Schneidkante hat.

## Revendications

1. Outil médical (2) de suture et de ligature comprenant :
un élément (20) flexible de suture et de ligature adapté pour suturer ou ligaturer un tissu biologique ; et
un élément (100) de maintien comportant une partie de maintien adaptée pour maintenir l'élément (20) de suture et de ligature dans un trajet le long duquel un élément (10) coupant adapté pour couper l'élément (20) de suture et de ligature se déplace,
**caractérisé en ce que**
l'élément (100) de maintien comporte en outre :
un premier élément (101) formé dans une forme cylindrique, dans lequel au moins un trou latéral (103a, 103b) est formé sur le premier élément (101) ; et
un deuxième élément (102) formé dans une forme cylindrique, le deuxième élément (102) étant monté sur le premier élément (101) de façon à couvrir une périphérie externe du premier élément (101) et destiné à pouvoir coulisser sur la périphérie externe du premier élément (101) dans une direction longitudinale du premier élément (101),
dans lequel une longueur du deuxième élément (102) dans la direction longitudinale est plus courte qu'une longueur d'une ouverture du trou latéral (103a, 103b) dans la direction longitudinale, et
dans lequel le deuxième élément (102) est disposé sur une partie intermédiaire du trou latéral (103a, 103b) dans la direction longitudinale et l'élément (20) de suture et de ligature est inséré à travers à la fois une partie du trou latéral (103a, 103b) plus près d'un côté distinct du premier élément (101) que le deuxième élément (102) et une partie du trou latéral (103a, 103b) plus près d'un côté proximal du premier élément (101) que le deuxième élément (102).

2. Outil médical (2) de suture et de ligature selon la revendication 1, dans lequel
le premier élément (101) comporte une surface en fuseau (104, 105) en regard du trou latéral (103a, 103b), et
la surface en fuseau (104, 105) est formée de façon à augmenter la longueur de l'ouverture du trou latéral (103a, 103b) dans la direction longitudinale allant de la périphérie externe du premier élément (101) à une périphérie interne du premier élément (101).

3. Instrument médical (1) de suture et de ligature comprenant :
l'outil médical (2) de suture et de ligature selon la revendication 1 ou 2 ; et
une unité d'actionnement (3), dans laquelle l'outil médical (2) de suture et de ligature est attaché de manière détachable sur une extrémité distale de l'unité d'actionnement (3), dans lequel
l'unité d'actionnement (3) comprend :
une section d'insertion (4) adaptée pour être insérée dans un corps, la section d'insertion (4) comportant une extrémité distale, une extrémité proximale et un axe allongé ; et
l'élément coupant (10) prévu de façon à être mobile par rapport à la section d'insertion (4), l'élément coupant comportant un bord coupant.
